# FASCICULE DE BREVET EUROPEEN

(11) **EP 3 116 565 B1**
(45) Date de publication et mention de la délivrance du brevet: **25.04.2018**
(21) Numéro de dépôt: 15718512.5
(22) Date de dépôt: 10.03.2015
(51) Int. Cl.: A61M 5/14, A61B 18/04, A61M 5/142, A61M 5/44

(54) **DISPOSITIF POUR GÉNÉRER DE LA VAPEUR À INJECTER DANS UN VAISSEAU HUMAIN OU ANIMAL**
VORRICHTUNG ZUR ERZEUGUNG VON DAMPF ZUR INJEKTION IN EIN MENSCHLICHES ODER TIERISCHES GEFÄSS
DEVICE FOR GENERATING VAPOUR FOR INJECTION INTO A HUMAN OR ANIMAL VESSEL

(30) Priorité: 11.03.2014 FR 1400584
(43) Date de publication de la demande: 18.01.2017
(73) Titulaire: MIRAVAS, 25000 Besançon (FR)
(72) Inventeur: RAUBER, Nicolas, 25000 Besançon (FR)
(74) Mandataire: GIE Innovation Competence Group
(86) Numéro de dépôt international: PCT/FR2015/050587
(87) Numéro de publication internationale: WO 2015/136211

(56) Documents cités:
- WO-A1-99/02112
- WO-A1-02/069821
- WO-A1-2009/149456
- US-A1- 2006 184 091

## Description

L'invention concerne un dispositif pour assurer le traitement thermique de pathologies veineuse par injection thermo-régulée de vapeur.

### Arrière plan de l'invention

Plus particulièrement, l'invention concerne un dispositif pour injecter de la vapeur générée à partir d'un liquide tel que de l'eau ou du liquide physiologique dans un vaisseau animal ou humain afin de le réchauffer, typiquement dans le but qu'il s'obture en se contractant. Concrètement, lorsque la vapeur atteint l'intérieur du vaisseau, elle se condense, et libère alors sa chaleur sensible et sa chaleur latente de condensation, ce qui permet de réchauffer efficacement le vaisseau en question.

Ce type de traitement est mis en oeuvre par exemple pour traiter des varices des hémorroïdes, des verrucosités ou autres : le praticien utilise alors un cathéter, ou bien une aiguille qu'il connecte à une pièce à main. Cette pièce à main est pourvue de moyens de chauffage et elle est reliée à une unité d'injection d'eau froide, qui s'apparente à une pompe, et qui fournit de manière pulsée de petites quantités de liquide, ayant un volume de l'ordre de quatre-vingt microlitres.

En fonctionnement, le cathéter est introduit dans le vaisseau à traiter, et l'unité d'injection introduit, à intervalles réguliers, des pulses de liquide froid dans la pièce à main qui comporte des moyens de chauffage. Chaque volume de liquide est vaporisé par les moyens de chauffage, et est ainsi diffusé dans le vaisseau via le cathéter qui est connecté de manière étanche à cette pièce à main.

Un tel appareillage, qui est décrit dans le document de brevet FR2915872, comporte une pièce à main capable de chauffer assez rapidement chaque pulse d'eau qu'elle reçoit de l'unité d'injection.

En pratique, un tel appareillage est nécessairement sophistiqué, de sorte qu'il ne peut être disponible qu'en milieu hospitalier, un tel système est trop coûteux pour qu'un praticien puisse l'acquérir en vue de pratiquer ce type de traitement dans son cabinet médical. Les documents WO-A-99/02112, US-A-2006/184091, WO-A-2009/149456 et WO-A-02/069821 décrivent des pièces à main selon le préambule de la revendication 1.

### Objet de l'invention

Le but de l'invention est de proposer une solution pour remédier à cet inconvénient en proposant un appareillage d'injection de vapeur ayant un coût réduit.

### Résumé de l'invention

A cet effet, l'invention a pour objet une pièce à main pour injecter de la vapeur dans un vaisseau animal ou humain afin de réchauffer ce vaisseau, cette pièce à main comprenant des moyens pour générer de la vapeur à partir d'un liquide tel que de l'eau ou du liquide physiologique et un organe de connexion à un cathéter ou à une aiguille à introduire dans le vaisseau pour y injecter la vapeur, pièce à main dans laquelle les moyens de génération de vapeur comportent :
- un réservoir destiné à contenir le liquide, ce réservoir étant équipé de moyens de chauffage électriques, pour chauffer le liquide afin de le vaporiser et de produire un flux de vapeur se diffusant via l'organe de connexion, et
- une vanne de sortie (9) anti-retour interposée entre le réservoir (6) et l'organe de connexion (8) pour interdire le passage de fluide depuis l'organe de connexion (8) vers le réservoir (6), la dite vanne de sortie (9) étant une vanne de régulation de pression (9) qui s'ouvre pour laisser passer la vapeur depuis le réservoir (6) vers le connecteur (8) lorsque la pression dans le réservoir (6) a atteint une valeur seuil.

Grâce à cette pièce à main, la vapeur est produite sans unité d'injection : tout le liquide est directement présent dans le réservoir et il est entièrement réchauffé dans la pièce à main. Comme une pompe n'est plus nécessaire, l'appareillage est moins coûteux et donc accessible pour un cabinet médical. Selon la réalisation de la vanne de sortie et selon les moyens de chauffage électriques, le flux de vapeur peut se diffuser de manière continue ou pas, comme on le verra mieux par la suite.

Les moyens de chauffage sont avantageusement situés à l'intérieur du réservoir pour être entourés par le liquide et/ou la vapeur à réchauffer, ce qui permet de minimiser les déperditions thermiques : la chaleur produite par effet Joule irradie directement le mélange de liquide et de vapeur que contient le réservoir. Dans un exemple, les moyens de chauffage (7) comportent une résistance électrique.

Avantageusement, le réservoir est prérempli au moins partiellement de liquide à vaporiser. Ainsi, il n'est pas nécessaire d'apporter du liquide au cours d'un traitement.

La pièce à main selon l'invention peut comporter également des moyens de remplissage du réservoir incluant un clapet anti-retour assurant que du liquide et/ou de la vapeur ne peut pas quitter le réservoir via les moyens de remplissage, en particulier pendant l'utilisation de la pièce à main.

La pièce à main peut comprendre en outre une vanne de purge du réservoir ; ceci permet de vider l'air contenu dans le réservoir avant chauffage.

Selon un mode de réalisation de l'invention, la vanne de sortie est une vanne de régulation de pression à un seuil ; ainsi, en régime établi, la pièce à main peut produire un flux de vapeur continu, comme on le verra mieux plus loin. Selon un autre mode de réalisation, adapté pour la production d'un flux de vapeur pulsé, la vanne de sortie est une vanne de régulation de pression à deux seuils :
- la vanne de sortie s'ouvrant pour laisser passer la vapeur lorsque la pression dans le réservoir atteint un seuil haut, et
- la vanne de sortie se fermant pour empêcher la vapeur de passer lorsque la pression dans le réservoir devient inférieure à un seuil bas inférieur au seuil haut.

L'invention concerne également un système comprenant une pièce à main telle que définie ci-dessus, et une unité de pilotage destinée à être connectée à cette pièce à main pour alimenter électriquement ses moyens de chauffage, cette unité de pilotage comprenant des moyens pour limiter la puissance de l'alimentation électrique des moyens de chauffage.

Dans un tel système, la pièce à main peut comporter en outre une sonde thermique située dans le réservoir, et l'unité de pilotage peut comporter des moyens d'asservissement de la puissance électrique d'alimentation des moyens de chauffage sur la température délivrée par la sonde thermique et / ou sur la température des moyens de chauffage, de manière à asservir la puissance électrique d'alimentation des moyens de chauffage à une température maximale admissible dans le réservoir.

Le système peut comprendre également un cathéter pourvu d'une vanne interdisant le passage de fluide depuis le cathéter vers le réservoir, par exemple une vanne de type anti-retour et/ou une vanne s'ouvrant lorsque la pression dans le réservoir atteint une valeur seuil.

### Brève description des figures

L'invention sera mieux comprise, et d'autres caractéristiques et avantages de l'invention apparaîtront à la lumière de la description qui suit d'exemples de pièces à main et de systèmes selon l'invention. Ces exemples sont donnés à titre non limitatif. La description est à lire en relation avec les dessins annexés dans lesquels :
- La figure 1 est une représentation schématique d'un système d'injection de vapeur conforme à l'invention.
- La figure 2 est une représentation schématique d'une pièce à main conforme à l'invention.

### Description d'un mode de réalisation de l'invention

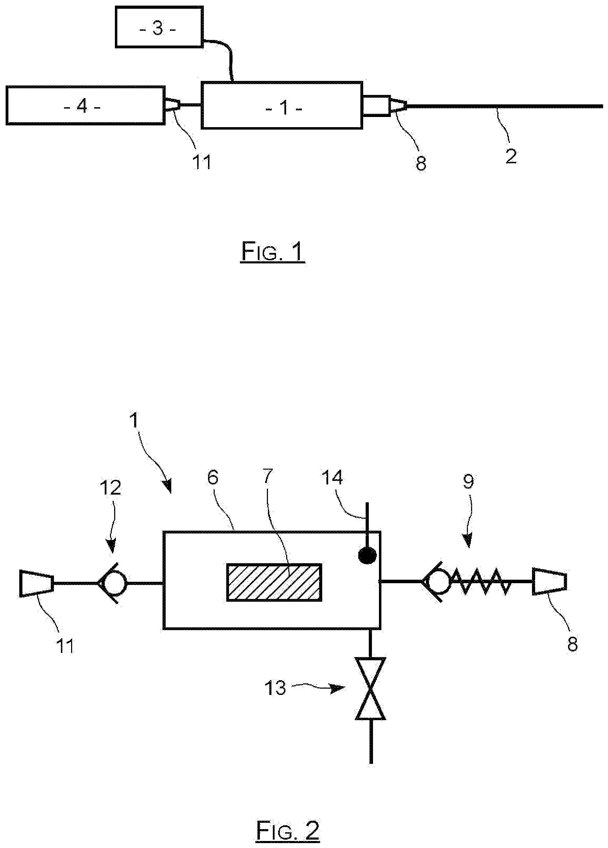

Le système selon l'invention qui est représenté schématiquement à la figure 1 comporte une pièce à main 1 pour générer de la vapeur en chauffant un liquide, afin de diffuser cette vapeur via un cathéter 2 connecté à cette pièce à main, et une unité de pilotage 3 alimentée par un réseau électrique et qui est elle-même électriquement connectée à la pièce à main 1 pour l'alimenter électriquement. Dans une variante (non représentée), l'unité de pilotage 3 est alimentée par un accumulateur ou une batterie d'accumulateurs.

La pièce à main 1 qui est représentée schématiquement dans les figures par un rectangle, a en fait une forme ergonomique permettant de manière aisée sa préhension et sa manipulation par un praticien la tenant dans sa main.

Dans la configuration de la figure 1, un récipient 4 est connecté à la pièce à main 1 pour la remplir avec un liquide tel que de l'eau stérile ou bien un sérum physiologique devant être chauffé pour produire la vapeur pressurisée à injecter. Comme visible de manière plus détaillée dans la figure 2, la pièce à main 1 comporte un réservoir chauffant 6, comprenant une enveloppe par exemple en matière plastique rigide, renfermant un élément chauffant électrique 7 de type résistance électrique qui permet de réchauffer le contenu du réservoir par effet Joule.

L'élément chauffant 7 est situé à l'intérieur du réservoir 6 qu'il équipe, par exemple dans sa région centrale, de sorte que la surface externe de cet élément 7 irradie la chaleur produite par effet Joule vers le contenu du récipient, à savoir du liquide et/ou de la vapeur. Les déperditions thermiques sont ainsi minimisées puisque toute la chaleur issue de l'élément 7 réchauffe en premier lieu le contenu du réservoir, de manière à générer rapidement de la vapeur.

Cet élément chauffant peut avoir une forme hélicoïdale, droite, tubulaire, ou autre, et il peut être fabriqué avec différents matériaux, tels que un acier inoxydable, un Inconel ®, ou tout autre matériau approprié. A titre d'ordre de grandeur, pour l'application envisagée, sa résistance électrique est comprise entre un dixième et quatre ohm, et elle vaut par exemple un ohm.

La pièce à main 1 est équipée d'un connecteur dédié, repéré par 8, de type standardisé, destiné à recevoir un cathéter 2 pour connecter de manière étanche ce cathéter au réservoir 1 dans lequel la vapeur est produite. Ce connecteur standardisé est ainsi apte à recevoir tout cathéter couramment disponible en milieu médical. Ce connecteur est relié par une tubulure non représentée à une région interne du réservoir permettant d'y recueillir de la vapeur plutôt que du liquide : le fluide arrivant dans le connecteur 8 est ainsi par exemple collecté en partie haute du réservoir 6, ou au niveau de sa connexion au réservoir.

Grâce au connecteur à cathéter, le praticien choisit le cathéter adapté au traitement qu'il doit réaliser avant de le connecter à la pièce à main 1 pour procéder à ce traitement. Le cathéter utilisé est prévu pour supporter une température élevée, par exemple cent-cinquante degrés, et il a des dimensions adaptées pour être introduit dans le vaisseau qui doit être traité.

Comme représenté schématiquement dans l'exemple de la figure 2, la pièce à main 1 intègre également une vanne de sortie 9 qui est interposée entre l'enveloppe interne que délimite le réservoir 6, et le connecteur 8 recevant le cathéter. Cette vanne de sortie 9 est une vanne de régulation de pression : elle s'ouvre lorsque la pression dans le réservoir 6 atteint une certaine valeur pour libérer la vapeur présente dans le réservoir 6 vers le cathéter. Cette vanne de sortie 9 reste fermée tant que la pression dans le réservoir 6 est insuffisante. Du sang ne peut ainsi pas refluer depuis le vaisseau traité vers le réservoir de la pièce à main dans lequel est générée la vapeur.

Dans l'exemple représenté schématiquement à la figure 2, cette vanne de sortie 9 a une structure générale correspondant à celle d'un clapet anti-retour à bille équipé d'un ressort qui est taré à une certaine valeur. Cette vanne 9 s'ouvre ainsi lorsque l'écart de pression entre son entrée et sa sortie est supérieur à une valeur seuil correspondant à la valeur de tarage du ressort, et elle reste fermée sinon. Lors de l'activation des moyens de chauffage, le liquide est chauffé ce qui augmente la pression dans le réservoir. Lorsque la pression atteint une valeur seuil, la vanne s'ouvre, la vapeur s'échappe du réservoir. En régime établi, stable, si le liquide est chauffé en continu, le débit de vapeur de la vanne s'ajuste pour maintenir la pression à la valeur seuil dans le réservoir. Lors le chauffage est coupé, la vanne se ferme, une dépression se crée à l'intérieur du réservoir, dépression qui entraîne un remplissage du réservoir par aspiration du liquide contenu dans le récipient 4.

En pratique, cette vanne de sortie 9 permet ainsi de réguler la pression dans le réservoir 6 à une valeur prédéterminée qui correspond de fait à une valeur de température de la vapeur générée dans le réservoir. Par exemple, pour l'eau, une vapeur à cent-dix degrés correspond à une pression de quatorze dixièmes de bars dans le réservoir; une vapeur à cent-vingt degrés correspond à une pression de deux bars dans le réservoir ; et une vapeur à cent-cinquante degrés correspond à une pression de cinq bars dans le réservoir.

Autrement dit, en limitant la pression dans le réservoir 6, la vanne de sortie 9 permet de piloter également la température dans le réservoir, attendu qu'en fonctionnement, la pression dans le réservoir 6 est la pression de vapeur saturante du mélange de liquide et de vapeur. Le tarage de la vanne de sortie 9 conditionne ainsi directement la température de la vapeur produite.

On notera que si ce tarage est proche de zéro, la pression se stabilise à un bar dans le réservoir 6, ce qui donne lieu à la production d'une vapeur à cent degrés en sortie de vanne 9. La vanne 9 assure alors uniquement une fonction de clapet anti-retour.

Cette vanne 9 est dimensionnée et tarée lors de sa fabrication, par exemple pour s'ouvrir lorsque la pression dans le réservoir 6 atteint deux bars, de manière à libérer une vapeur ayant une température proche de cent-vingt degrés.

Dans une variante (non représentée), la vanne de sortie 9 est une vanne de régulation de pression à deux seuils :
- la vanne de sortie s'ouvre pour laisser passer la vapeur lorsque la pression dans le réservoir atteint un seuil haut, et
- la vanne de sortie se ferme pour empêcher la vapeur de passer lorsque la pression dans le réservoir devient inférieure à un seuil bas inférieur au seuil haut.

Ainsi, en régime établi, la vanne reste fermée de manière cyclique pendant le temps nécessaire pour que la pression dans le réservoir passe du seuil bas (correspondant à la fermeture de la vanne) au seuil haut (correspondant à l'ouverture de la vanne). La pièce à main produit ainsi un flux de vapeur pulsé. La quantité de vapeur produite à chaque pulse et l'intervalle de temps entre deux pulses sont ajustés en ajustant l'énergie thermique fournie par les moyens de chauffage ainsi que les seuils haut et bas de la vanne 9.

Dans l'exemple des figures, la vanne 9 est intégrée à la pièce à main, mais il est également possible de prévoir qu'elle équipe le cathéter connecté à cette pièce à main, en vue de produire le même résultat.

Dans l'exemple des figures, la pièce à main 1 comporte en outre un connecteur de remplissage 11, d'un format avantageusement standardisé, et par l'intermédiaire duquel est assuré le remplissage du réservoir avec le liquide approprié au traitement. Comme visible dans la figure 2, la pièce à main 1 est pourvue d'un clapet anti-retour 12 interposé entre son réservoir 6 et le connecteur de remplissage 11 de sorte que le contenu du réservoir 6 ne peut pas refluer vers le récipient 4 contenant le liquide de remplissage.

Une autre possibilité peut consister à prévoir des moyens de remplissage comportant une ouverture équipée d'un bouchon vissable pour fermer de manière étanche cette ouverture de remplissage du réservoir une fois que le remplissage est terminé.

Ce clapet anti-retour 12 permet ainsi, le cas échéant, de conserver le récipient 4 connecté à la pièce à main 1 durant le traitement, ce qui permet de remplir à nouveau le réservoir 6 au fur et à mesure que le traitement est réalisé. Il est également possible de placer le récipient 4 en hauteur, celui-ci pouvant alors être une poche contenant le liquide qui est alors suspendue à un support. Dans ce cas le réservoir peut être alimenté à peu près continuellement et naturellement par la poche sous l'effet de la gravité s'exerçant sur le liquide qu'elle contient, de manière à remplir le réservoir de la pièce à main au fur et à mesure qu'il se vide.

La pièce à main 1 peut encore être équipée d'une vanne de purge 13 permettant de relier l'intérieur du réservoir 6 à l'environnement extérieur. Cette vanne de purge 13 est par exemple actionnée manuellement pour chasser une partie de la vapeur ou du gaz présent à l'intérieur du réservoir 6 lors ou après remplissage de ce dernier.

Hormis les éléments électriquement conducteurs, les composants de la pièce à main 1, comme en particulier son réservoir 6, sont fabriqués en matière plastique résistant à la chaleur, selon un procédé de moulage par injection ou autre, de manière à présenter un faible coût de production. Cette pièce à main 1 peut ainsi avantageusement être prévue à usage unique.

A ce titre, la pièce à main 1 peut être produite dans différentes dimensions correspondant chacune à un volume (une capacité) de réservoir, à choisir en fonction des dimensions de la veine ou du vaisseau à traiter. Le praticien choisit alors une pièce à main dont le réservoir a une capacité d'autant plus élevée que la veine à traiter a de grandes dimensions.

La pièce à main 1 est alimentée électriquement par l'unité de pilotage 3 qui délivre dans la résistance chauffante 7 une puissance électrique permettant d'assurer la vaporisation du liquide contenu dans le réservoir 6. La quantité de chaleur introduite dans la pièce à main 1 peut ainsi être estimée et régulée directement depuis l'unité 3 en suivant dans cette unité la puissance électrique qui est effectivement consommée dans la pièce à main 1.

On peut ainsi éviter un accroissement trop important de la température dans le réservoir simplement en contrôlant la tension et/ou l'intensité du courant avec lequel l'unité 3 alimente la pièce à main 1. L'unité 3 peut ainsi être exploitée pour limiter la puissance électrique injectée dans la pièce à main 1 afin d'éviter que la température ne devienne trop élevée dans le réservoir 6. On constate dans la pratique que les échanges thermiques entre l'élément chauffant et la vapeur sont moins bons qu'entre l'élément chauffant et le liquide. Ceci se traduit par un risque de surchauffe dans le réservoir, risque qui s'accroît au fur et à mesure que la quantité de liquide diminue dans le réservoir.

Pour améliorer le contrôle de la température et l'état du mélange se trouvant dans le réservoir 6, ce réservoir est avantageusement équipé d'une sonde thermique 14 qui mesure la température et qui est connectée à l'unité 3. L'intensité du courant (ou de la tension ou de la puissance) d'alimentation de la pièce à main 1 peut ainsi être limitée par une consigne de température maximale à ne pas dépasser. L'unité 3 peut ainsi augmenter l'intensité si la température est inférieure à la consigne, et la diminuer sinon.

Par exemple, la vanne de régulation 9 est tarée à deux bars pour libérer la vapeur lorsqu'elle atteint deux bars et donc cent-vingt degrés, et l'unité de pilotage 3 est configurée pour réguler l'alimentation électrique de manière à ce que la température dans le réservoir ne puisse pas excéder cent-vingt-cinq, ou bien cent-trente degrés.

Ainsi, dans le cas où pour une raison ou une autre, la vapeur ne s'évacue pas correctement par le cathéter, la température de la pièce à main ne peut pas augmenter inconsidérément, et les paramètres (température et pression) de production de la vapeur ne sont pas perturbés de manière significative.

A l'inverse, lors du début du chauffage du liquide, qui est alors à température ambiante, l'unité de commande peut alors augmenter significativement la puissance électrique tant que la température nominale n'est pas atteinte, de manière à accélérer le début de la chauffe et provoquer plus rapidement le début de la vaporisation du liquide.

D'une manière générale, l'unité 3 est prévue pour être connectée à un réseau d'alimentation électrique, et à la pièce à main 1 afin de l'alimenter électriquement en basse tension, par exemple en courant continu sous douze volts et selon une puissance électrique de l'ordre de cent-cinquante watt.

L'unité 3 est avantageusement pourvue d'une interface avec un écran et de différents éléments de contrôle permettant au praticien de limiter la température maximale, de visualiser la température effective dans le réservoir, ainsi que la puissance électrique consommée dans la pièce à main 1.

Comme on l'aura compris, la pièce à main 1 est équipée d'un connecteur électrique par l'intermédiaire duquel elle est électriquement branchée à l'unité de pilotage 3 pour d'une part recevoir le courant d'alimentation de sa résistance électrique, et d'autre part transférer les valeurs de température mesurée par la sonde thermique 14 vers l'unité 3.

En ce qui concerne le connecteur standardisé recevant le cathéter, il s'agit avantageusement d'un connecteur de type Luer-Lock, qui correspond à un standard très communément utilisé en milieu médical, et permettant par là-même la connexion à de multiples gammes de cathéters. Il en va de même du connecteur de remplissage du réservoir 6 lorsqu'il est prévu, qui est alors lui aussi avantageusement du type Luer-Lock.

L'utilisation du système selon l'invention par un praticien se déroule comme suit. Dans un premier temps, il choisit le cathéter 2 à utiliser compte tenu de la pathologie à traiter et il le connecte à la pièce à main, après quoi il remplit le réservoir 6 avec le liquide et choisit grâce à l'unité 3 l'énergie à délivrer pour traiter la veine.

Plus concrètement, l'unité 3 est prévue pour afficher, par exemple en temps réel, la quantité de chaleur qui a été transférée via la vapeur véhiculée par le cathéter, vers le vaisseau traité. Ce calcul est par exemple réalisé à partir d'abaques mémorisées dans l'unité 3 résultant d'une campagne d'essais, et permettant de connaître pour différentes valeurs de puissance électrique consommée par les moyens de chauffage, la puissance thermique effectivement transférée dans un vaisseau en cours de traitement.

Le praticien peut alors introduire le cathéter 2 dans le vaisseau à traiter. Une fois que le cathéter est en place, il active l'unité 3 pour débuter le chauffage et la vaporisation du liquide contenu dans le réservoir 6. Le chauffage s'opère alors pour atteindre une température nominale prédéterminée valant par exemple cent-vingt degrés, qui peut être, comme indiqué plus haut conditionnée uniquement par le tarage de la vanne de sortie 9 régulant la pression dans le réservoir.

Lorsqu'une durée suffisante est écoulée, le liquide commence à se vaporiser dans le réservoir 6, jusqu'à atteindre la pression suffisante pour ouvrir la vanne de régulation 9. Cette ouverture provoque alors le début de la diffusion de la vapeur vers le cathéter, et donc vers le vaisseau en cours de traitement.

Le praticien continue de maintenir le cathéter en place, et il peut éventuellement le déplacer le long du vaisseau pour répartir l'apport de chaleur tout au long de la portion à traiter.

Une fois que la quantité de chaleur suffisante a été apportée, le praticien peut retirer le cathéter et considérer que le traitement est terminé. Si la contenance du réservoir est inférieure au volume de liquide à vaporiser pour la quantité de chaleur devant être apportée, le praticien peut alors remplir à nouveau le réservoir de manière à poursuivre le traitement jusqu'à ce que la quantité de chaleur suffisante ait été apportée.

Dans l'exemple d'utilisation ci-dessus, c'est le praticien qui remplit la pièce à main 1 avec le volume de liquide approprié pour le traitement à réaliser. Mais la pièce à main 1 peut être à usage unique prévue pré-remplie de liquide, et disponible dans différentes tailles correspondant chacune à une capacité volumétrique du réservoir qu'elle comporte. Il suffit alors au praticien de choisir la pièce à main 1 ayant la taille adaptée au traitement à réaliser (c'est-à-dire ayant le volume de liquide approprié). Le praticien utilise alors la pièce à main 1 qu'il a choisie pour réaliser le traitement, et il la jette une fois que le traitement est terminé. L'invention est telle que définie dans les revendications annexées.

## Revendications

1. Pièce à main (1) pour injecter de la vapeur dans un vaisseau animal ou humain afin de réchauffer ce vaisseau, cette pièce à main comprenant des moyens pour générer de la vapeur à partir d'un liquide tel que de l'eau ou du liquide physiologique et un organe de connexion (8) à un cathéter (2) ou à une aiguille à introduire dans le vaisseau pour y injecter la vapeur, les moyens de génération de vapeur comportant :
- un réservoir (6, 7) destiné à contenir le liquide, ce réservoir (6) étant équipé de moyens de chauffage électriques (7), pour chauffer le liquide afin de le vaporiser et de produire un flux de vapeur se diffusant via l'organe de connexion (8), et **caractérisé en ce que** les moyens de génération de vapeur comportent également :
- une vanne de sortie (9) anti-retour interposée entre le réservoir (6) et l'organe de connexion (8) pour interdire le passage de fluide depuis l'organe de connexion (8) vers le réservoir (6), la dite vanne de sortie (9) étant une vanne de régulation de pression (9) qui s'ouvre pour laisser passer la vapeur depuis le réservoir (6) vers le connecteur (8) lorsque la pression dans le réservoir (6) a atteint une valeur seuil.

2. Pièce à main selon la revendication 1, comportant des moyens de remplissage du réservoir (6) incluant un clapet anti-retour (12) assurant que du liquide et/ou de la vapeur ne peut pas quitter le réservoir (6) via les moyens de remplissage.

3. Pièce à main selon l'une des revendications 1 à 2, dans laquelle les moyens de chauffage (7) comportent une résistance électrique située à l'intérieur du réservoir (6) pour être entourée par le liquide et/ou la vapeur à réchauffer.

4. Pièce à main selon l'une des revendications 1 à 3, dont le réservoir (6) est prérempli au moins partiellement de liquide à vaporiser.

5. Pièce à main selon l'une des revendications 1 à 4, comportant en outre une vanne de purge (13) du réservoir (6).

6. Pièce à main selon l'une des revendications 1 à 5, dans laquelle la vanne de sortie (9) est une vanne de régulation de pression à deux seuils :
- la vanne de sortie s'ouvrant pour laisser passer la vapeur lorsque la pression dans le réservoir atteint un seuil haut, et
- la vanne de sortie se fermant pour empêcher la vapeur de passer lorsque la pression dans le réservoir devient inférieure à un seuil bas inférieur au seuil haut.

7. Système comportant une pièce à main selon l'une des revendications 1 à 6, et une unité de pilotage (3) destinée à être connectée à cette pièce à main (1) pour alimenter électriquement ses moyens de chauffage (7), cette unité de pilotage (3) comprenant des moyens pour limiter la puissance de l'alimentation électrique des moyens de chauffage (7).

8. Système selon la revendication 7, dans lequel la pièce à main (1) comporte en outre une sonde thermique (14) située dans le réservoir (6), et dans lequel l'unité de pilotage (3) comporte des moyens d'asservissement de la puissance électrique d'alimentation des moyens de chauffage (7) sur la température délivrée par la sonde thermique (14) et / ou sur la température des moyens de chauffage pour asservir la puissance électrique d'alimentation des moyens de chauffage (7) à une température maximale admissible dans le réservoir (6).

9. Système comportant une pièce à main selon l'une des revendications 7 à 8 et un cathéter, et dans lequel ce cathéter est pourvu d'une vanne interdisant le passage de fluide depuis le cathéter vers le réservoir (6), sous forme d'une vanne de type antiretour et/ou d'une vanne s'ouvrant lorsque la pression dans le réservoir (6) atteint une valeur seuil.

## Patentansprüche

1. Handstück (1) zur Injektion von Dampf in ein tierisches oder menschliches Gefäß, um dieses Gefäß zu erwärmen, wobei dieses Handstück Mittel zur Erzeugung von Dampf aus einer Flüssigkeit, wie Wasser oder einer physiologischen Flüssigkeit, und ein Verbindungselement (8) mit einem Katheter (2) oder mit einer Nadel umfasst, die in das Gefäß einzuführen sind, um den Dampf in dieses zu injizieren, wobei die Mittel zur Erzeugung von Dampf umfassen:
- einen Behälter (6, 7), der dazu bestimmt ist, die Flüssigkeit zu enthalten, wobei dieser Behälter (6) mit elektrischen Heizmitteln (7) zum Erwärmen der Flüssigkeit ausgestattet ist, um diese zu verdampfen und einen Dampfstrom zu erzeugen, der über das Verbindungselement (8) diffundiert,
und **dadurch gekennzeichnet, dass** die Mittel zur Erzeugung von Dampf auch umfassen:
- ein Auslassrückschlagventil (9), das zwischen dem Behälter (6) und dem Verbindungselement (8) angeordnet ist, um den Durchgang von Fluid aus dem Verbindungselement (8) in den Behälter (6) zu unterbinden, wobei das Auslassventil (9) ein Druckregelventil (9) ist, das sich öffnet, um den Dampf aus dem Behälter (6) in den Verbinder (8) strömen zu lassen, wenn der Druck im Behälter (6) einen Schwellenwert erreicht.

2. Handstück nach Anspruch 1, umfassend Mittel zur Füllung des Behälters (6), welche eine Rückschlagklappe (12) aufweisen, die sicherstellt, dass Flüssigkeit und/oder Dampf den Behälter (6) über die Füllmittel nicht verlassen kann.

3. Handstück nach einem der Ansprüche 1 bis 2, wobei die Heizmittel (7) einen elektrischen Widerstand umfassen, der im Inneren des Behälters (6) angeordnet ist, um von der Flüssigkeit und/oder dem Dampf, die zu erwärmen sind, umgeben zu sein.

4. Handstück nach einem der Ansprüche 1 bis 3, wobei der Behälter (6) mindestens teilweise mit zu verdampfender Flüssigkeit vorgefüllt ist.

5. Handstück nach einem der Ansprüche 1 bis 4, ferner umfassend ein Ablassventil (13) des Behälters (6).

6. Handstück nach einem der Ansprüche 1 bis 5, wobei das Auslassventil (9) ein Druckregelventil mit zwei Schwellen ist:
- wobei sich das Auslassventil öffnet, um den Dampf hindurchgehen zu lassen, wenn der Druck im Behälter eine hohe Schwelle erreicht, und
- wobei sich das Auslassventil schließt, um zu verhindern, dass der Dampf hindurchgeht, wenn der Druck im Behälter niedriger wird als eine tiefe Schwelle, die niedriger ist als die hohe Schwelle.

7. System, umfassend ein Handstück nach einem der Ansprüche 1 bis 6, und eine Steuereinheit (3), die dazu bestimmt ist, mit diesem Handstück (1) verbunden zu werden, um seine Heizmittel (7) elektrisch zu versorgen, wobei diese Steuereinheit (3) Mittel zur Begrenzung der Leistung der elektrischen Versorgung der Heizmittel (7) umfasst.

8. System nach Anspruch 7, wobei das Handstück (1) ferner eine Wärmesonde (14) umfasst, die im Behälter (6) angeordnet ist, und wobei die Steuereinheit (3) Mittel zur Regelung der elektrischen Leistung der Versorgung der Heizmittel (7) gemäß der von der Wärmesonde (14) abgegebenen Temperatur und/oder gemäß der Temperatur der Heizmittel umfasst, um die elektrische Leistung der Versorgung der Heizmittel (7) auf eine zulässige maximale Temperatur im Behälter (6) zu regeln.

9. System, umfassend ein Handstück nach einem der Ansprüche 7 bis 8, und einen Katheter, und wobei dieser Katheter versehen ist mit einem Ventil, das den Durchgang von Fluid aus dem Katheter in den Behälter (6) unterbindet, in der Form eines Ventils vom Rückschlagtyp und/oder eines Ventils, das sich öffnet, wenn der Druck im Behälter (6) einen Schwellenwert erreicht.

## Claims

1. Handpiece (1) for injecting steam into a human or animal vessel in order to heat this vessel, this handpiece comprising means for generating steam from a liquid such as water or physiological liquid and a connection member (8) for connecting to a catheter (2) or to a needle that is to be introduced into the vessel in order to inject the steam into same, the steam-generating means comprising:
- a reservoir (6, 7) intended to contain the liquid, this reservoir (6) being equipped with electrical heating means (7) for heating the liquid in order to vaporize it and produce a stream of steam that diffuses via the connection member (8), and **characterized in that** the steam-generating means also comprise:
- a nonreturn outlet valve (9) interposed between the reservoir (6) and the connection member (8) for preventing fluid from passing from the connection member (8) toward the reservoir (6), said outlet valve (9) being a pressure-regulating valve (9) which opens to allow steam to pass from the reservoir (6) toward the connector (8) when the pressure in the reservoir (6) has reached a threshold value.

2. Handpiece according to Claim 1, comprising means for filling the reservoir (6) including a nonreturn valve (12) ensuring that liquid and/or steam cannot leave the reservoir (6) via the filling means.

3. Handpiece according to either of Claims 1 and 2, in which the heating means (7) comprise an electrical resistance situated inside the reservoir (6) so as to be surrounded by the liquid and/or the steam that is to be heated.

4. Handpiece according to one of Claims 1 to 3, of which the reservoir (6) is at least partially pre-filled with liquid that is to be vaporized.

5. Handpiece according to one of Claims 1 to 4, further comprising a purge valve (13) for purging the reservoir (6).

6. Handpiece according to one of Claims 1 to 5, in which the outlet valve (9) is a two-threshold pressure-regulating valve:
- the outlet valve opening to allow the steam to pass when the pressure in the reservoir reaches a high threshold, and
- the outlet valve closing to prevent the steam from passing when the pressure in the reservoir drops below a low threshold lower than the high threshold.

7. System comprising a handpiece according to one of Claims 1 to 6 and a control unit (3) intended to be connected to this handpiece (1) to supply heating means (7) with electricity, this control unit (3) comprising means for limiting the power of the electrical supply to the heating means (7).

8. System according to Claim 7, in which the handpiece (1) further comprises a thermal probe (14) situated inside the reservoir (6), and in which the control unit (3) comprises servo control means for controlling the power of the electrical supply to the heating means (7) on the basis of the temperature delivered by the thermal probe (14) and/or on the basis of the temperature of the heating means so as to servo control the electrical power supplied to the heating means (7) to a maximum permissible temperature in the reservoir (6).

9. System comprising a handpiece according to either of Claims 7 and 8 and a catheter, and in which this catheter is provided with a valve that prevents fluid from passing from the catheter toward the reservoir (6), in the form of a valve of the nonreturn type and/or of a valve that opens when the pressure in the reservoir (6) reaches a threshold value.
